# EUROPÄISCHE PATENTSCHRIFT

(11) **EP 1 259 206 B1**
(45) Veröffentlichungstag und Bekanntmachung des Hinweises auf die Patenterteilung: **28.12.2005**
(21) Anmeldenummer: 01923623.1
(22) Anmeldetag: 02.03.2001
(51) Int. Cl.: A61F 13/15, A61L 15/42, A61L 15/00

(54) **ABSORBIERENDE STRUKTUR SOWIE VERFAHREN ZU DEREN HERSTELLUNG**
ABSORBENT STRUCTURE AND METHOD FOR PRODUCING THE SAME
STRUCTURE ABSORBANTE ET SON PROCEDE DE PRODUCTION

(30) Priorität: 02.03.2000 DE 10010269; 02.03.2000 DE 10010268
(43) Veröffentlichungstag der Anmeldung: 27.11.2002
(73) Patentinhaber: Paul Hartmann Aktiengesellschaft, 89522 Heidenheim (DE)
(72) Erfinder: MALOWANIEC, D., Krzysztof, 89522 Heidenheim (DE); MANGOLD, Rainer, 89542 Herbrechtingen (DE); WURSTER, Thomas, 89522 Heidenheim (DE)
(74) Vertreter: Friz, Oliver
(86) Internationale Anmeldenummer: PCT/EP2001/002387
(87) Internationale Veröffentlichungsnummer: WO 2001/064155

(56) Entgegenhaltungen:
- WO-A-94/13460
- WO-A-98/56430
- DE-A- 2 222 780
- US-A- 5 061 259
- US-A- 5 859 077
- US-A- 6 019 871

## Beschreibung

Die Erfindung betrifft eine auf Basis körniger superabsorbierender Polymermaterialien gebildete absorbierende Struktur, wobei die superabsorbierenden Polymermaterialien durch ein niedriger schmelzendes thermoplastisches Polymer miteinander verbunden sind. Desweiteren betrifft die Erfindung ein Verfahren zum Herstellen einer derartigen absorbierenden Struktur und einen Hygieneartikel mit einer solchen absorbierenden Struktur als eine Saugkörperlage.

Wenn vorstehend von einer auf Basis superabsorbierender Polymermaterialien gebildeten absorbierenden Struktur die Rede ist, so wird hierunter eine Struktur mit einem Anteil von mehr als 70 Gewichtsprozent an superabsorbierenden Polymermaterialien verstanden. Es entspricht ständigem Verständnis und ständiger Übung auf dem hier interessierenden Gebiet absorbierender Strukturen, dass unter superabsorbierenden Materialien solche Materialien verstanden werden, die durch Absorption oder Gelbildung wenigstens etwa das zehnfache ihrer eigenen Masse an Flüssigkeit aufzunehmen und dauerhaft zu halten vermögen. Dabei wird die Flüssigkeit in die molekulare Struktur dieser Materialien eingebunden und nicht etwa nur in Poren der Materialien aufgenommen, aus denen sie wieder herausgepresst werden könnte. Derzeitige superabsorbierende Materialien sind wasserunlösliche vernetzte Polymere, die in der Lage sind, unter Quellung und Ausbildung von Hydrogelen wässrige Flüssigkeiten und Körperflüssigkeiten wie Urin und Blut aufzunehmen und die absorbierter Flüssigkeitsmenge zumindest unterhalb eines gewissen Umgebungsdrucks dauerhaft zurückzuhalten.

Es hat sich gezeigt, dass Saugkörperstrukturen mit einem derart hohen Gehalt, also mehr als 70 Gew.-%, an superabsorbierenden Polymermaterialien (SAP) mit herkömmlichen auf Fasern basierenden Strukturen nicht realisierbar sind, da die körnigen SAP-Materialien einerseits nicht hinreichend zugänglich angeordnet und andererseits nicht hinreichend immobilisiert werden können.

Aus DE-A-2 222 780 ist es bekannt, zur Herstellung einer auf Basis superabsorbierender Polymermaterialen gebildeten absorbierenden Struktur die körnigen superabsorbierenden Polymermaterialien zusammen mit Teilchen eines thermoplastischen Werkstoffs auf eine Unterlage aufzubringen und den thermoplastischen Werkstoff dann zu erschmelzen, um einen Verbund zu erhalten.

Eine derartige Struktur aus superabsorbierenden Polymermaterialien und thermoplastischen Polymeren konnte sich aber in der Praxis nicht durchsetzen, da die Zugänglichkeit der superabsorbierenden Materialien für die auftreffende Flüssigkeit nicht hinreichend gewährleistet war. Zudem erwies sich diese Struktur als zu starr und zeichnete sich daher durch einen ungenügenden Tragkomfort aus.

Aus WO 94/13460 ist die Herstellung eines offenzelligen Polypropylenschaums mit einem Porenanteil von mehr als 20 Vol.-% bekannt. Als Anwendungsgebiete sind Verpackungszwecke und die Verwendung des Schaums zur Schallabsorption und thermischen Isolation genannt.

Aus WO 98/56430 ist ebenfalls die Herstellung eines extrudierten thermoplastischen Schaums bekannt. Der Schaum hat vorzugsweise eine aus Zellwänden und Hohlräumen bestehende Struktur. Der Schaum kann beschreibungsgemäß als Aufnahmekörper für ein Stück Fleisch oder als Lage einer Windel verwendet werden.

Der Erfindung liegt die Aufgabe zugrunde, bei einer absorbierenden Struktur der eingangs genannten gattungsgemäßen Art mit hohem SAP-Gehalt die vorstehenden Nachteile zu beseitigen, d.h. eine flexible Struktur mit guter Flüssigkeitsaufnahme und -speichercharakteristik, die zudem einfach hergestellt werden kann, zu erreichen.

Diese Aufgabe wird durch eine gattungsgemäße absorbierende Struktur gelöst, die erfindungsgemäß dadurch hergestellt ist, dass superabsorbierende körnige Polymermaterialien mit einem Feuchtigkeitsgehalt von wenigstens 0,5 Gew.-% bezogen auf die Gesamtmasse der superabsorbierenden Polymermaterialien und das thermoplastische Polymer extrudiert werden und dabei die Flüssigkeit der superabsorbierenden Polymermaterialien verdampft und dadurch eine Schäumung der Struktur herbeigeführt wird.

Mit der vorliegenden Erfindung wird vorgeschlagen, körniges, partikelförmiges superabsorbierendes Polymermaterial durch zumindest teilerschmolzene thermoplastische Polymerwerkstoffe zu verbinden, indem die unter Druck und Temperatur stehende Mischung unter Anwesenheit eines Treibmittels, welches von dem Feuchtegehalt der superabsorbierenden Materialien gebildet ist, extrudiert wird. Hierdurch kann einerseits eine Immobilisierung, d.h. eine Fixierung der körnigen superabsorbierenden Polymermaterialien innerhalb der Struktur erreicht werden, und andererseits wird eine Struktur gebildet, welche von einer auftreffenden wässrigen Flüssigkeit, wie z. B. Urin, sehr gut durchdrungen werden kann. Es zeigte sich, dass die Flüssigkeit sehr rasch in die durch Extrusion des Gemischs unter Expansion des Treibmittels, also durch Verdampfen der in den superabsorbierenden Polymermaterialien enthaltenen Flüssigkeit gebildete offenporige geschäumte Struktur eindringen und zu den darin aufgenommenen superabsorbierenden Polymermaterialien gelangen kann, wo sie dann dauerhaft gespeichert wird. Es zeigte sich auch, dass die quellfähigen superabsorbierenden Polymermaterialien in der erfindungsgemäßen Struktur in weit geringerem Maße den Effekt des sogenannten Gelblockings verursachen, welcher sich bei höheren gewichtsprozentualen Anteilen von quellfähigen Polymermaterialien in absorbierenden Faserstrukturen als problematisch erweist, weil die in der Flüssigkeit quellenden Polymermaterialien die Faserzwischenräume zusammendrücken, so dass keine Kapillarität zum Flüssigkeitstransport in noch ungenutzte Saugkörperbereiche mehr zur Verfügung steht. Ein weiteres Problem bei aus natürlichen Fasern gebildeten Saugkörperstrukturen ist deren Neigung im eingenässten Zustand in sich zusammenzufallen, welches Problem auch als wet-collaps bezeichnet wird. Auch dies führt zu einer Reduzierung des Flüssigkeitsverteilungsvermögens innerhalb einer absorbierenden Struktur. Bei der erfindungsgemäßen extrudierten absorbierenden Struktur treten die vorstehend erörterten Probleme nicht oder in weit geringerem Maße auf, weshalb das Absorptionsvermögen der superabsorbierenden Polymermaterialien auch bei sehr hohen Konzentrationen von mehr als 70 Gew.% nahezu vollständig zur Aufnahme der auftreffenden Flüssigkeit zur Verfügung steht.

Dadurch, dass als Treibmittel bei der Extrusion der Mischung aus superabsorbierenden Polymermaterialien und thermoplastischem Polymer der Feuchtigkeitsgehalt, also die Flüssigkeit, welche die wenigstens 0,5 Gew.-% Feuchtigkeit der superabsorbierenden Polymermaterialien ausmachen, verwendet wird, lässt sich ein für die Absorptionsfähigkeit der superabsorbierenden Polymermaterialien in der fertigen Struktur maßgeblicher Effekt erzielen: Durch das schlagartige Verdampfen der Flüssigkeit aus den körnigen superabsorbierenden Polymermaterialien beim Extrudieren wird die Oberfläche dieser Körner und das sich an die Oberfläche der Körner anlegende thermoplastische Polymer nach allen Richtungen aufgerissen. Dieses Aufreißen bewirkt eine hervorragende Zugänglichkeit der körnigen superabsorbierenden Polymermaterialien, und es wird verhindert, dass sich das erschmolzene thermoplastische Polymer schmelzenartig über die Oberfläche der Körner legt und damit einen Flüssigkeitszutritt behindert. Durch diese erfindungsgemäße Maßnahme, nämlich feuchte superabsorbierende Polymermaterialien zur Extrusion zu verwenden, konnte die Zugänglichkeit der körnigen superabsorbierenden Polymermaterialien für Flüssigkeit weiter verbessert werden.

Die Korngröße der Partikel aus superabsorbierenden Materialien liegt im üblichen Bereich und beträgt im Massenmittel vorzugsweise etwa 200 - 800 µm, wobei vorzugsweise nicht mehr als 20 Masse-% der Partikel kleiner als 200 µm sind; es wird diesbezüglich auf die Offenbarung in der US-Patentschrift 5,061,259 verwiesen.

Vorzugsweise ist die erfindungsgemäße absorbierende Struktur hergestellt unter Verwendung eines superabsorbierenden Polymermaterials mit einem Feuchtigkeitsgehalt von wenigstens 1 Gew.-% und besonders bevorzugtermaßen von wenigstens 4 Gew.-%. Es versteht sich, dass ein möglichst hoher Feuchtigkeitsgehalt angestrebt wird, wobei dies durch die mit steigendem Feuchtigkeitsgehalt schlechter werdende Handhabbarkeit der körnigen superabsorbierenden Polymermaterialien begrenzt ist. Die Erfindung schließt auch nicht aus, dass zusätzlich zur Verwendung von superabsorbierenden Polymermaterialien mit einem Feuchtigkeitsgehalt im angegebenen Bereich zusätzlich ein Treibmittel, beispielsweise in Form von CO₂ verwendet wird.

Dies ist indessen - wie sich überraschenderweise herausstellte - nicht erforderlich, um den Anwendungen genügende Retentionskapazitäten für Flüssigkeit zu erhalten.

Die extrudierte offenporige Struktur weist eine Retentionskapazität von wenigstens 10g Flüssigkeit je Gramm der extrudierten Struktur auf. Die Absorptionskapazität kann in einem noch näher zu beschreibenden Testverfahren bestimmt werden.

In weiterer Ausbildung der Erfindung ist der masseprozentuale Anteil des thermoplastischen Polymers geringer als 30 Gew.-%, insbesondere 20 Gew.%, und insbesondere geringer als 10 Gew.% der absorbierenden Struktur.

Als thermoplastisches Polymer, welches quasi das Verbindungsmittel der superabsorbierenden körnigen Polymermaterialien bildet, hat sich in besonders bevorzugter Weise ein Polymer aus der Gruppe der Polyolefine, insbesondere Polypropylen und/oder Polyethylen, erwiesen. Auch entsprechende Copolymere, insbesondere Ethylenvinylacetatcopolymere sowie halogenierte Polyolefine sind verwendbar. Grundsätzlich sind jedoch auch andere thermoplastischer Polymere zur Herstellung der erfindungsgemäßen absorbierenden Struktur geeignet, z.B. solche aus der Gruppe der Styrolpolymere.

Um ein möglichst großes Flüssigkeitsaufnahmevolumen zur Verfügung zu stellen und eine möglichst große Oberfläche der superabsorbierenden Polymermaterialien zur Flüssigkeitsaufnahme zu exponieren, liegt der Schäumungsgrad bei wenigstens 20%, vorzugsweise ist er höher, insbesondere 20 - 50% oder darüber. Der Schäumungsgrad bzw. der Begriff der "Schäumung" der Struktur ist dabei definiert oder wird verstanden als die Volumenzunahme einer Masseeinheit der Mischung im Zustand innerhalb der Extrusionsvorrichtung einerseits bzw. im extrudierten Zustand der fertigen Struktur andererseits.

In vorteilhafter Weise kann die absorbierende Struktur zwischen 3 und 20, vorzugsweise zwischen 5 und 10 Gew.% Fasern als Zuschlagstoffe umfassen. Hierbei kann es sich um natürliche oder synthetische Fasern, vorzugsweise Polyesterfasern, handeln, deren Schmelz- oder Zersetzungstemperatur aber höher ist als die Schmelztemperatur des verwandten thermoplastischen Polymers innerhalb der Extrusionsvorrichtung. Die Fasern bewirken, daß beim Extrusionsvorgang Kanäle gebildet werden, die das Eindringen von wässriger Flüssigkeit in die Struktur fördern.

Die Erfindung ermöglicht in besonders vorteilhafter Weise, dass absorbierende Strukturen gebildet werden können, deren Flächengewicht in Längsrichtung und/oder in Querrichtung der Struktur variiert, wobei die Längsrichtung mit der Extrusionsrichtung übereinstimmt. Durch eine entsprechende Gestaltung einer Extrusionsöffnung, insbesondere eines Extrusionsschlitzes, lassen sich an sich beliebige Querschnittsstrukturen erzielen. So könnte insbesondere im Querschnitt senkrecht zur Längsrichtung betrachtet die Dicke der absorbierenden Struktur mittig größer sein und entsprechend der Struktur der Extrusionsöffnung zu den Seiten hin in beliebiger Weise abnehmen.

Wie auch alle nachfolgend zu erläuternden absorbierenden Strukturen kann die Struktur außerdem eine oberflächenaktive Substanz, insbesondere ein Hydrophilisierungsmittel zu einem Anteil von vorzugsweise 0,2 - 10% umfassen. Die bereits extrudierte Struktur kann sekundär mit dem Hydrophilisierungsmittel beaufschlagt werden. Vorzugsweise wird dieses Mittel aber gemeinsam mit den übrigen Ausgangsstoffen dem Extruder zugeführt oder in die bereits erschmolzene Polymermasse injiziert, sie befindet sich also bereits in Mischung mit der Polymerschmelze bevor diese extrudiert wird.

Vorteilhafterweise werden hierfür Alkylsulfonate, Fettsäurederivate oder Fluorchemikalien - wie Sie in der Veröffentlichung "Polymer Melt Additives: Their Chemistry Structure and Uses", (Autoren Gasper et al. Vortrag während der Insight 1999 - Nonwovens Business/Fiber & Fabric Conferences, San Diego, California, 1-2 November 1999. Proceedings herausgegeben durch Marketing Technology Services, Inc.) beschrieben sind - eingesetzt.

Mit der vorliegenden Erfindung wird auch Schutz beansprucht für, einen absorbierenden Hygieneartikel zum einmaligen Gebrauch, insbesondere eine Windel, eine Damenbinde oder eine Inkontinenzvorlage, mit einem insbesondere mehrschichtigen Saugkörper, der gekennzeichnet ist durch eine Saugkörperlage aus einer absorbierenden Struktur der vorstehend beschriebenen erfindungsgemäßen Art.

Diese Saugkörperlage kann auf der körperabgewandten Seite einer Flüssigkeitsverteiler- und Zwischenspeicherschicht angeordnet sein. Es ist auch denkbar, dass die Flüssigkeitsverteiler- und Zwischenspeicherschicht, die weniger oder überhaupt keine superabsorbierenden Polymermaterialien umfasst, ebenfalls als extrudierte geschäumte Struktur, insbesondere unter Zusatz eines Treibmittels, wie z.B. CO₂, hergestellt ist. Solchenfalls könnten beide Saugkörperlagen innerhalb der Herstellungsmaschine durch Extrusion hergestellt und zur Bildung des Schichtenverbunds übereinandergelegt werden. Auch eine unmittelbare Coextrusion beider Schichten, d.h. Herstellung durch dieselbe Extrusionsvorrichtung, ist denkbar und vorteilhaft.

Es ist darüber hinaus weiterhin möglich, die erfindungsgemäße SAP-haltige Struktur selbst mehrschichtig auszubilden. So kann z.B. eine erste körperabgewandte Schicht von einer zweiten körperzugewandten Schicht überlagert sein. Solchenfalls kann z.B. die absorbierende SAP-haltige Struktur mit einem vorteilhaften SAP-Profil ausgestattet werden. Insbesondere kann die erste körperabgewandte Schicht weniger SAP (in Gew.-% bezogen auf diese erste Schicht) enthalten als die zweite körperzugewandte Schicht. Dabei kann es vorteilhaft sein, dass die flächenhafte Erstreckung, d.h. Breite und/oder Länge, der ersten körperabgewandten Schicht verschieden ist von der flächenhaften Erstreckung der zweiten körperzugewandten Schicht, insbesondere kann es vorteilhaft sein, die erste körperabgewandte Schicht hinsichtlich ihrer flächenhaften Erstreckung größer, insbesondere breiter auszubilden als die zweite körperzugewandte Schicht. Auch dieser mehrschichtige Aufbau der absorbierenden SAP-haltigen Struktur selbst lässt sich einfach durch unmittelbare Coextrusion der Schichten herstellen.

Des weiteren wäre es denkbar, daß eine körperabgewandte flüssigkeitsundurchlässige Schicht, die üblicherweise von einer vorgefertigten Kunststoff-Folie gebildet ist, durch Coextrusion mit der Saugkörperlage hergestellt ist. In diesem Fall würde es sich als vorteilhaft und zweckmäßig erweisen, alle drei vorerwähnten Schichten oder gar noch weitere Schichten durch Coextrusion mittels einer einzigen Extrusionsvorrichtung innerhalb der Herstellungsmaschine auszubilden. Es kann dann vorteilhafterweise auf ein Fixiermittel, wie zum Beipiel ein Heißschmelzkleber, verzichtet werden, da die extrudierten Schichten untereinander aber auch gegenüber weiteren Lagen und/oder Elementen im Zuge ihrer Herstellung fixiert werden können.

Es wird generell angemerkt, daß auch die Flüssigkeitsverteiler- und Zwischenspeicherschicht, welche sehr wenig oder überhaupt keine superabsorbierenden Polymermaterialien enthalten kann, im übrigen so ausgebildet und hergestellt werden kann, wie die erfindungsgemäße absorbierende Struktur bzw. die vorerwähnte Saugkörperlage. Sie kann also Zuschlagstoffe in Form von Fasern oder oberflächenaktive Substanzen aufweisen und beispielsweise mit variierender Dicke bzw. variierendem Flächengewicht hergestellt ausgebildet sein.

Wie bereits vorstehend angedeutet kann es sich als vorteilhaft erweisen, wenn der Saugkörper in Längsrichtung des Artikels oder in Querrichtung des Artikels eine variierende Dicke aufweist, d.h. wenn er profiliert ausgebildet ist. Durch eine Materialanhäufung in einem mittleren Bereich des Hygieneartikels kann demzufolge dort die zur Verfügung stehende Flüssigkeitsabsorptionskapazität mit an sich beliebigem Profil, insbesondere gaussförmig oder stufenförmig, gebildet werden.

Es ist aber in ganz besonders vorteilhafter Ausbildung der Erfindung auch möglich, dass die Saugkörperlage beidseits in Längsrichtung des Artikels verlaufende und in Richtung auf den Benutzer emporstehende Wandabschnitte aufweist, die eine Auslaufsperre bilden. Diese Wandabschnitte übernehmen dann die Funktion von in Richtung auf den Benutzer emporstehenden Bündchenelementen, die bei bekannten Hygieneartikeln üblicherweise aus Vliesstoffen mit eingebrachten Elastifizierungsmitteln gebildet sind.

Es versteht sich, dass derartige Wandabschnitte auch in Querrichtung des Artikels verlaufen können und auch dort eine Sperrwirkung, insbesondere zum Trennen von festen und flüssigen Körperausscheidungen, ausüben können.

Gegenstand der vorliegenden Erfindung ist auch ein Verfahren zum Herstellen einer absorbierenden Struktur, insbesondere nach den Ansprüchen 1 bis 12 mit folgenden Verfahrensschritten:
- Einbringen eines thermoplastischen Polymers in eine Extrusionsvorrichtung,
- Einbringen eines superabsorbierenden körnigen Polymermaterials mit einem Feuchtigkeitsgehalt von wenigstens 0,5, insbesondere wenigstens 1 Gew.-%, insbesondere von wenigstens 4 Gew.-%, in die Extrusionsvorrichtung,
- Schmelzen des thermoplastischen Polymermaterials bei Temperaturen unterhalb einer Schmelz- oder Zersetzungstemperatur des superabsorbierenden Polymermaterials,
- Extrudieren des Gemischs, wobei die Flüssigkeit in dem superabsorbierenden Polymermaterial bei Druckabbau verdampft und zur Schäumung des thermoplastischen Polymers führt, welches die körnigen Polymermaterialien miteinander matrixbildend verbindet.

Als Treibmittel bei der Extrusion wird also die in den superabsorbierenden Polymermaterialien enthaltene Feuchte verwendet. Es wäre denkbar, zusätzlich ein Treibmittel, wie CO₂ zu verwenden, obschon dies nicht erforderlich ist. Auch gesättigte, ungesättigte, cyclische Kohlenwasserstoffe und halogenierte Kohlenwasserstoffe sowie Edelgase wie Argon, Helium, oder Stickstoff oder ein Wasser/Luft-Gemisch wären denkbar.

Es wird innerhalb der Extrusionsvorrichtung ein solcher Überdruck aufgebaut, dass die Flüssigkeit der feuchten superabsobierenden Polymermaterialien, die bei der Extrusion als Treibmittel dient, beim Durchtritt durch die Extrusionsöffnung verdampft.

Wenn zusätzlich CO₂ als Treibmitel verwendet wird, wird es im sogenannten überkritischen Zustand bei Temperaturen oberhalb von etwa 31°C und Drücken oberhalb von etwa 73,5 bar zugeführt. In diesem Zustand lässt sich das Treibmittel optimal zur Vorbereitung eines physikalischen Schäumungsvorgangs mit den superabsorbierenden Polymermaterialien und mit dem geschmolzenen thermoplastischen Polymer vermischen. Wird diese Mischung dann durch eine Extrusionsöffnung in einen Bereich niedrigeren Drucks gegeben, so verdampft das Treibmittel bei abnehmender Temperatur, und es entsteht durch Volumenzunahme die geschäumte offenporige Struktur.

Da aber nicht nur ein hinreichender Druck für die Flüssigkeit bzw. die Feuchte in den superabsorbierenden Polymermaterialien bzw. für das Treibmittel erreicht werden muss, sondern auch das thermoplastische Polymer zumindest teilerschmolzen werden muss, werden innerhalb der Extrusionsvorrichtung Temperaturen von 80 bis 200 °C geschaffen.

Zur Herstellung von in Längs- und/oder Querrrichtung variierender Dicke oder Gestalt der herzustellenden Struktur wird der Extrusionsquerschnitt während des Extrudierens verändert. Wenn eine große Anzahl von entsprechend ausgebildeten Strukturen extrudiert werden soll, so erweist es sich als vorteilhaft, wenn der Extrusionsquerschnitt entsprechend oszillierend verändert wird. Dies erfolgt quer zur Extrusionsrichtung, und zwar in der Ablegerichtung, wodurch die Dicke einer extrudierten Bahn variiert wird, oder quer zur Ablegerichtung, wodurch deren Breite variiert wird.

Um die Zugänglichkeit der extrudierten Struktur für wässrige Flüssigkeiten zu erhöhen, ist es vorteilhaft, die extrudierte Struktur einer weiteren mechanischen Behandlung, z.B. einer Streckung, einer Verpressung (Walzung) und/oder einer Perforierung durch ein feines Nadelwerkzeug auszusetzen.

Vorteilhaft ist insbesondere eine mehrstufige Walzung der extrudierten Struktur. Eine mehrstufige Walzung ermöglicht die Anwendung mehrerer Temperatur- und/oder Druckstufen. Damit kann die extrudierte Struktur gezielter hinsichtlich der Erfordernisse ihrer späteren Verwendung verändert/optimiert werden. So hat es sich als vorteilhaft erwiesen, die extrudierte Struktur in einer ersten Kalanderstufe bei einer Temperatur zu verpressen, die geeignet ist, das thermoplastische Polymer in der extrudierten Struktur oberhalb des Erweichungspunktes zu halten. Je nach verwendetem Polymer hat sich eine Temperatur in der Kalanderstufe von 40-90°C, insbesondere 50-60°C, als geeignet erwiesen. Vorteilhafterweise kann die extrudierte absorbierende Struktur anschließend in einer zweiten Kalanderstufe verpresst werden, die kalt, insbesondere bei Temperaturen von 0-30°C, insbesondere bei 15-25°C, durchgeführt wird.

Es hat sich ferner als vorteilhaft erwiesen, außerdem eine Verstreckung der extrudierten Struktur vorzunehmen.

Als ganz besonders vorteilhaft erweist es sich, wenn das erfindungsgemäße Verfahren in einen Herstellungsprozess für Hygieneartikel integriert wird und dabei eine Saugkörperlage unmittelbar innerhalb einer Maschine extrudiert wird. Solchenfalls kann auf Faserbildungs- und Ablegestationen bei der Herstellungsmaschine (zumindest für die extrudierte Saugkörperlage) verzichtet werden. Wie bereits erwähnt, können auch mehrere Saugkörperlagen, die übereinander anzuordnen sind, innerhalb derselben Maschine hergestellt werden.

Weitere Einzelheiten, Merkmale und Vorteile der Erfindung ergeben sich aus den beigefügte Patentansprüchen sowie aus der zeichnerischen Darstellung und nachfolgenden Beschreibung einer Herstellungsvorrichtung, des Herstellungsverfahrens sowie einiger Ausführungsformen erfindungsgemäßer absorbierender Strukturen. In der Zeichnung zeigt:
- Figur 1: eine schematische Ansicht einer Vorrichtung zum Herstellen einer erfindungsgemäßen absorbierenden Struktur;
- Figuren 2 bis 6: verschiedene Ausführungsformen erfindungsgemäßer absorbierender Strukturen;
- Figur 7: eine weitere Ausführungsform einer mehrschichtigen erfindungsgemäßen absorbierenden Struktur und
- Figur 8: eine schematische Darstellung einer Coextrusionsvorrichtung.

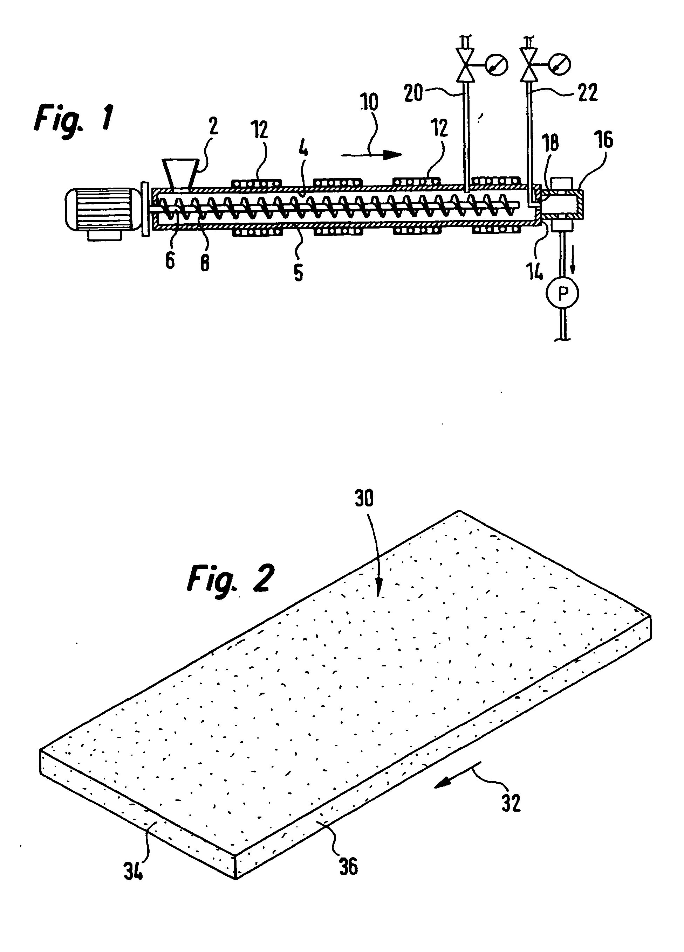

Figur 1 zeigt eine Vorrichtung zum Herstellen einer erfindungsgemäßen absorbierenden Struktur. Die Vorrichtung umfaßt eine trichterförmige Eingabeeinrichtung 2, über die ein Feststoffgemisch, das vorzugsweise zuvor gemäß der gewichtsprozentualen Zusammensetzung der einzelnen Bestandteile hergestellt wurde, in einen zylindrischen Innenraum 4 eines hochdruckstabilen rohrförmigen Gehäusekörpers 5 der Herstellungsvorrichtung eingegeben eingegeben werden kann. In diesen Innenraum 4 erstreckt sich eine elektromotorisch angetriebene Welle 6 mit einem wendelförmigen Schneckengang 8. Beim Antrieb der Welle 6 wird die eingebrachte Feststoffmischung weiter vermischt und in Längsrichtung 10 gefördert. Am Außenumfang des rohrförmigen Gehäuses 5 sind Heizeinrichtungen 12 vorgesehen.

An dem der Eingabeeinrichtung 2 gegenüberliegenden Ende des rohrförmigen Gehäuses 5 ist an dessen Stirnseite 14 ein Extrusionswerkzeug 16 montierbar. Das Extrusionswerkzeug 16 kommuniziert über eine Öffnung 18 an der Stirnseite 14 mit dem Innenraum 4 des rohrförmigen Gehäuses.

In den Innenraum 4 münden Injektionseinrichtungen 20, 22, wobei die letztere quasi innerhalb der Öffnung 18 mündet. Über die Injektionseinrichtungen 20, 22 kann ein unter Betriebsdruck stehendes Treibmittel in den Innenraum 4 eingebracht werden. Auf diese Weise kann im Innenraum 4 ein Betriebsdruck in Abhängigkeit des im Extrusionsvorgang verwandten Treibmittels, im allgemeinen oberhalb 70 bar, eingestellt und während des Extrusionsvorgangs aufrechterhalten werden. Es kann aber auch ohne Zuleiten eines externen Treibmittels ein Betriebsdruck auf die Mischung innerhalb der Vorrichtung ausgeübt werden, indem Kolbenmittel oder bewegbare Wandabschnitte vorgesehen werden.

Zur Herstellung einer erfindungsgemäßen absorbierenden Struktur kann beispielsweise als thermoplastisches Polymer ein Polyolefin, insbesondere ein Polypropylen- und/oder Polyäthylen-Granulat, verwendet werden. Dieses Granulat wird mit quellfähigen superabsorbierenden Polymermaterialien, die in Verbindung mit absorbierenden Schichten bei Hygieneartikeln hinreichend bekannt sind und daher nicht näher beschrieben zu werden brauchen, vermischt, wobei diese superabsorbierenden Polymermaterialien einen Feuchtigkeitsgehalt von wenigstens 0,5 Gew.-% aufweisen. Das so erhaltene Gemisch wird über die Eingabeeinrichtung 2 in den Innenraum 4 gegeben. Durch die Heizeinrichtungen 12 wird das Gemisch auf eine solche Betriebstemperatur gebracht, daß das thermoplastische Polymer schmilzt, die körnigen superabsorbierenden Polymermaterialien aber in keinster Weise in Mitleidenschaft gezogen werden.

Es wird dann im Innenraum 4 der Injektionsvorrichtung ein Betriebsdruck erzeugt, der zum Extrudieren des teilerschmolzenen Gemischs über das Extrusionswerkzeug 16 mit Wasser als Treibmittel geeignet ist.

Wird ein zusätzliches Treibmittel, beispielsweise CO₂, verwendet werden soll, so kann dieses über die erwähnten Injektionseinrichtungen 20, 22 in vorzugsweise überkritischem Zustand in den Innenraum 4 eingeleitet werden.

Beim Durchtritt der so erhaltenen Mischung durch die Extrusionsöffnung des Extrusionswerkzeugs 16 und durch den damit einhergehenden Druckabbau verdampft die Flüssigkeit, die in den superabsorbierenden Polymermaterialien enthalten ist, und gegebenenfalls zusätzliches Treibmittel, und die Mischung wird geschäumt, d. h. es bilden sich durch die expandierende, verdampfende Flüssigkeit miteinander kommunizierende Poren oder Hohlräume. Innerhalb dieser durch Erstarren des thermoplastischen Polymers gebildeten Hohlraumstruktur sind die körnigen superabsorbierenden Polymermaterialien ortsfest gebunden. Sie sind immobilisiert, wobei dennoch ihre Oberfläche durch das Expandieren und Entweichen der Feuchtigkeit und die dadurch gebildeten Hohlräume exponiert ist und zur Flüssigkeitsaufnahme zur Verfügung steht.

Figur 2 zeigt einen Abschnitt einer extrudierten absorbierenden Struktur 30, welche zu 80 Gew.% ein superabsorbierendes Polymermaterial mit einem Feuchtigkeitsgehalt von wenigstens 1 Gew.-% (bezogen auf die Masse des superabsorbierenden Polymermaterials) und zu 13 Gew.% ein thermoplastisches Polymer, nämlich Polyethylen (PE), sowie zusätzlich zu 7 Gew.% Polyesterfasern (PES) umfasst. Mit dem Pfeil 32 ist die Extrusionsrichtung bezeichnet, so daß die mit dem Bezugszeichen 34 gebildete Endfläche die Ebene senkrecht zur Extrusionsrichtung 32 darstellt. Die absorbierende Struktur 30 ist in Figur 2 exakt quaderförmig dargestellt; es wird darauf hingewiesen, daß durch einen Extrusionsvorgang nur eine im wesentlichen ebene Oberfläche erhalten werden kann, und daß auch bei exakt rechteckförmiger Extrusionsöffnung verrundete Kanten gebildet werden. Es wäre indessen möglich, eine in Extrusionsrichtung 32 endlose Bahn durch Längs- und Querschneiden mit exakt senkrecht zueinander verlaufenden Endflächen 34 und Längsseitenflächen 36 auszubilden.

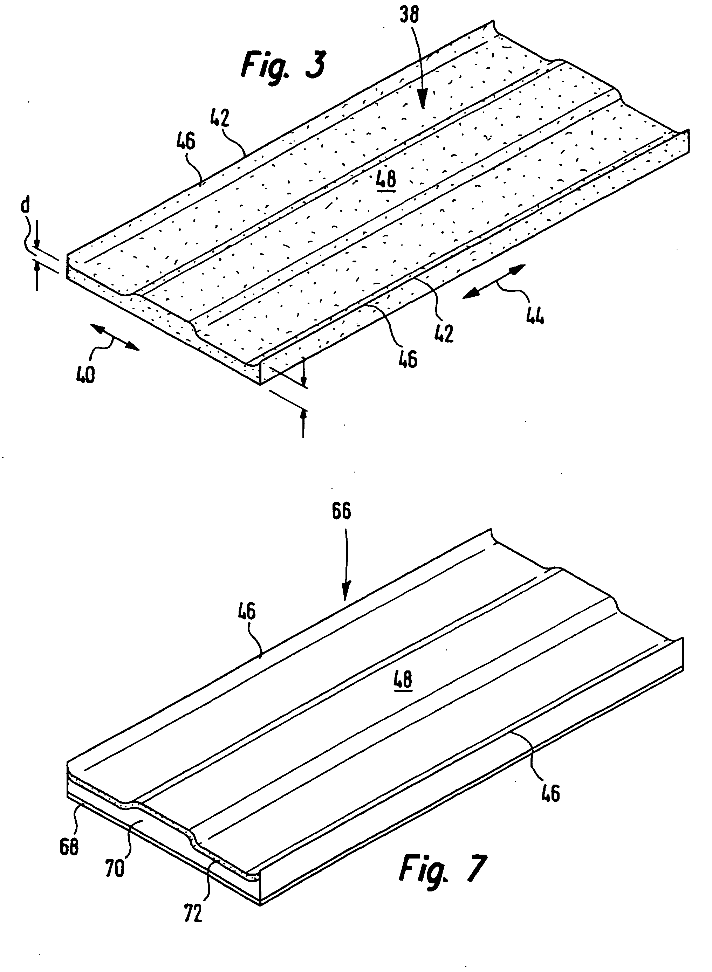

Figur 3 zeigt eine absorbierende Struktur 38, die in Querrichtung 40 eine variierende Dicke d aufweist. Die Struktur weist an ihren beiderseitigen Längsrändern 42 in Längsrichtung 44 verlaufend einen nach oben, also in Dickenrichtung emporstehenden Wandbereich 46 auf, der nach oben hin spitz ausläuft. Von außen nach innen, also in Querrichtung 40, fällt dieser Wandbereich 46 asymptotisch ab und geht in einen ebenen Abschnitt mit konstanter Dicke d über, um dann zur Mitte hin entsprechend dem aus Figur 3 ersichtlichen Profil zu einem Abschnitt 48 größerer Dicke wieder anzusteigen. Eine derartige Querschnittsstruktur läßt sich durch entsprechende Ausbildung des Extrusionsschlitzes herstellen.

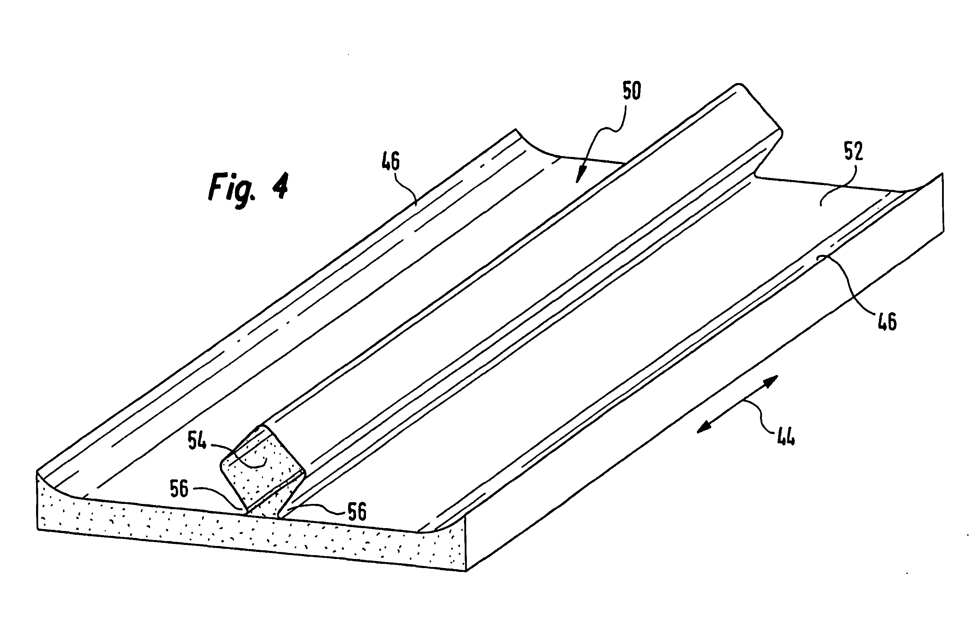

Figur 4 zeigt eine weitere Ausführungsform einer erfindungsgemäßen absorbierenden Struktur 50 mit entsprechend Figur 3 in Längsrichtung 44 verlaufenden emporstehenden beidseitigen Wandbereichen 46. Die Struktur 50 weist mittig einen ebenfalls in Längsrichtung 44 verlaufenden im Querschnitt im wesentlichen rautenförmigen und sich über einer Oberfläche 52 erhebenden Bereich 54 auf. Aufgrund seines rautenförmigen Querschnitts bildet der Bereich 54 in Richtung senkrecht zur Oberfläche 52 gesehen Hinterschnitte 56. Auch dies Ausbildung von im Querschnitt runden, elliptischen oder mehreckförmigen Strukturen mit oder ohne Hinterschnitte wäre denkbar. Derartige Saugkörperstrukturen sind zur Verwendung in Damenhygieneprodukten gedacht. Der erhabene Bereich 54, welche geometrische Form er auch immer haben mag, kann im Tragezustand zumindest teilweise in die Vagina eingreifen und somit einen direkten Kontakt zwischen Vagina und dem saugfähigen Hygieneprodukt herstellen.

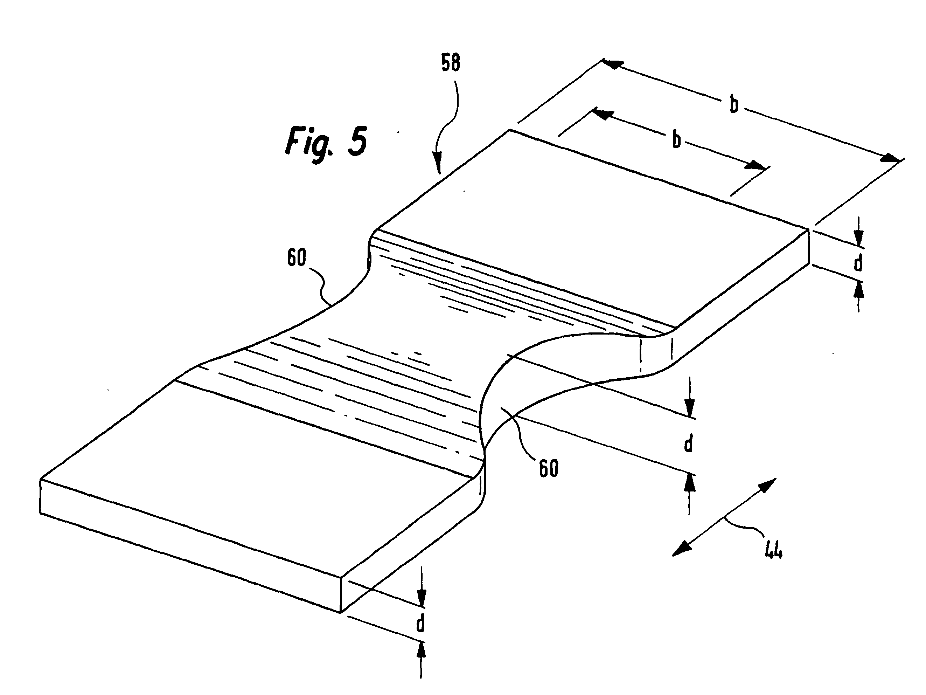

Figur 5 zeigt in entsprechender Ansicht eine durch Extrusion hergestellte absorbierende Struktur 58 mit in Längs- und Extrusionsrichtung 44 variierender Dicke d. Des weiteren weist die dargestellte absorbierende Struktur 58 in Längsrichtung 44 eine variierende Breite b auf. Die dargestellte absorbierende Struktur 58 würde sich zur Herstellung einer Windel eignen, wobei mittig bogenförmige Beinausschnitte 60 vorgesehen sind und in diesem den Schrittbereich der Windel bildenden Bereich eine Materialanhäufung durch die dort vorgesehene größere Dicke d gegeben ist.

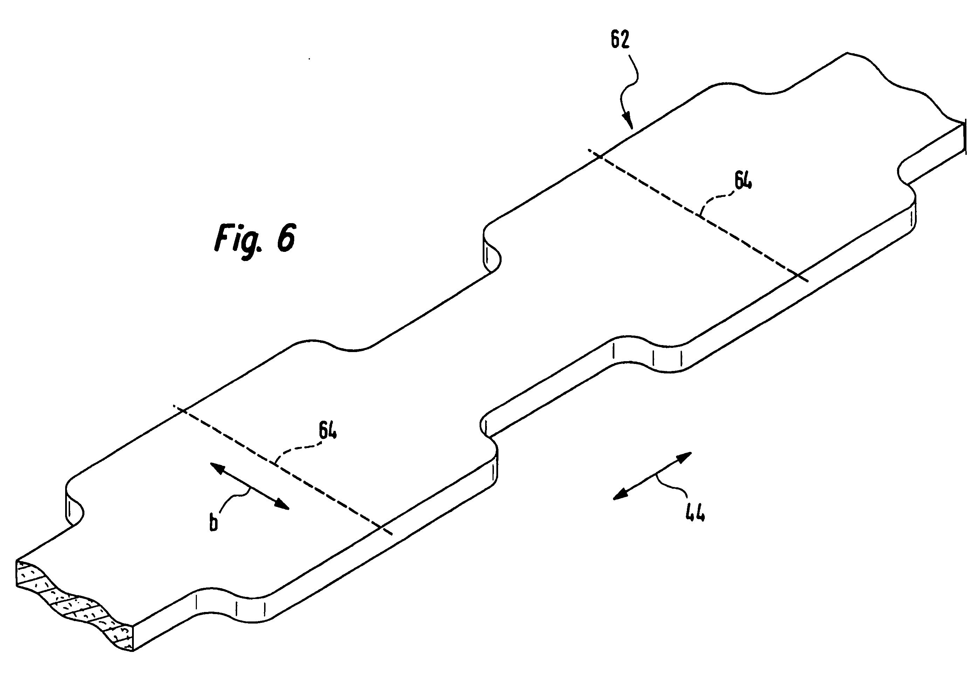

Figur 6 zeigt schematisch eine angedeutete endlose extrudierte Bahn 62 mit in Längs- und Extrusionsrichtung 44 variierender Breite b. Durch die unterbrochenen Linien 64 ist die Teilung der Endlosbahn durch Querschneiden zur Bildung einzelner Abschnitte für die Herstellung von Windeln angedeutet.

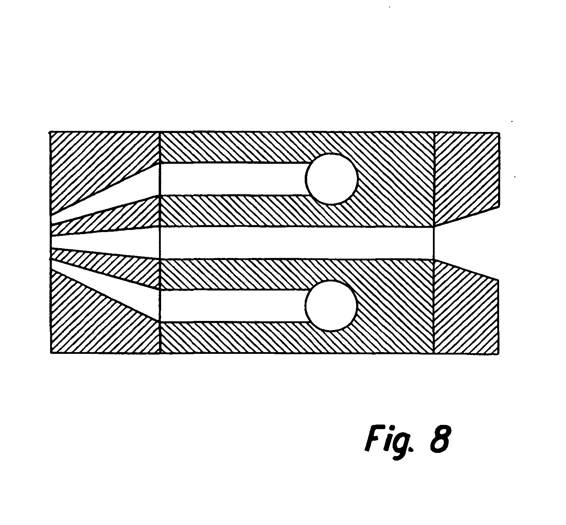

Figur 7 zeigt eine endlose extrudierte absorbierende Struktur 66, welche durch Coextrusion dreier Schichten hergestellt ist und sich für den Einsatz in einem Hygieneartikel, insbesondere einer Windel eignet. Die Struktur umfaßt eine erste untere extrudierte Folienschicht 68 aus PE und/oder PP. Eine mittlere auf Basis superabsorbierender Polymermaterialien mit einem Feuchtegehalt von wenigstens 1 Gew.-% gebildete extrudierte Schicht 70, die von der Zusammensetzung her der im Zusammenhang mit Figur 2 beschriebenen Schicht entsprechen kann, ist mit dem Bezugszeichen 70 bezeichnet. Auf deren Oberseite ist eine von superabsorbierenden Polymermaterialien freie Oberflächenschicht 72 auf Basis von Polyesterfasern (PES) einerseits und Polyethylen und/oder Polypropylen (PE/PP) andererseits vorgesehen. Alle drei Schichten 68, 70, 72 sind in einer Coextrusionsvorrichtung, wie sie schematisch in Figur 8 dargestellt ist, hergestellt, wobei zur Herstellung der Schicht 72 ein Treibmittel unter Überdruck eingesetzt wurde, um durch Expandieren und Verflüchtigen des Treibmittels eine offenporige geschäumte Struktur zu erzeugen. Die Struktur 66 ist im Querschnitt entsprechend Figur 3 ausgebildet; sie weist seitliche in Längsrichtung 44 verlaufende emporstehende Wandbereiche 46 auf, die in einem Hygieneartikel als Auslaufsperre dienen können und die Funktion von üblicherweise auf Basis von Vliesmaterialien gebildeten Bündchenelementen ausüben. Die Materialanhäufung durch eine größere Dicke der absorbierenden Schicht 70 in einem mittigen Bereich 48 stellt dort eine größere Flüssigkeitsabsorptionskapazität durch höhere Mengen an superabsorbierenden Polymermaterialien zur Verfügung. Die körperzugewandte obere Schicht 72 fungiert indessen als Flüssigkeitsverteiler- und Zwischenspeicherschicht. Dies bedeutet, sie erfaßt bei schwallartiger Flüssigkeitsbeaufschlagung eine große Menge von Flüssigkeit durch ihr großes Porenvolumen, um diese Flüssigkeit dann zeitverzögert in Dickenrichtung, aber auch in horizontaler Richtung zu verteilen und an die darunter befindliche Speicherschicht 70 abzugeben.

Das Flüssigkeitshaltevermögen einer erfindungsgemäßen extrudierten absorbierenden Struktur mit wenigstens 70 Gew.% Anteil an superabsorbierenden Polymermaterialien wird durch den nachfolgend zu beschreibenden Zentrifugentest durch Angabe des Retentionswerts bestimmt. Die zu untersuchende absorbierende Struktur wird im trockenen Zustand gewogen, um deren Masse in Gramm zu ermitteln. Es werden dann eine Anzahl von Prüflingen 30 Minuten lang vollständig in einer einprozentigen Natriumchlorid-Lösung von demineralisiertem Wasser als Prüflösung eingetaucht und anschließend 4 Minuten lang bei 276-facher Erdbeschleunigung geschleudert. Danach werden die Prüflinge wiederum gewogen, um die Masse einschließlich der darin gebundenen Flüssigkeit zu bestimmen. Die Masse der aufgenommenen oder gebundenen Flüssigkeit ergibt sich daher aus der Differenz der nach dem Schleudern bestimmten Masse und der Trockenmasse der jeweiligen Prüflinge. Dividiert man diese Differenz m_{fl} durch die Trockenmasse m_{trocken}, so erhält man den Retentionswert in der Einheit g_{fl}/g_{trocken}.

## Patentansprüche

1. Auf Basis superabsorbierender Polymermaterialien gebildete absorbierende Struktur (30, 38, 50, 58, 62, 66), wobei die superabsorbierenden Polymermaterialien durch ein thermoplastisches Polymer miteinander verbunden sind, dadurch hergestellt, dass superabsorbierende Polymermaterialien mit einem Feuchtigkeitsgehalt von wenigstens 0,5 Gew.-% bezogen auf die Gesamtmasse der superabsorbierenden Polymermaterialien und das thermoplastische Polymer extrudiert werden und dabei die Flüssigkeit der superabsorbierenden Polymermaterialien verdampft und eine Schäumung der Struktur herbeigeführt wird.

2. Absorbierende Struktur nach Anspruch 1 **dadurch gekennzeichnet, dass** superabsorbierende Polymermaterialien mit einem Feuchtigkeitsgehalt von wenigstens 1 Gew.-% zur Extrusion eingesetzt werden.

3. Absorbierende Struktur nach Anspruch 2 **dadurch gekennzeichnet, dass** superabsorbierende Polymermaterialien mit einem Feuchtigkeitsgehalt von wenigstens 4 Gew.-% zur Extrusion eingesetzt werden.

4. Absorbierende Struktur (30, 38, 50, 58, 62, 66), nach einem der vorhergehenden Ansprüche, **dadurch gekennzeichnet, dass** die Struktur eine Retentionskapazität von wenigstens 10 g/g aufweist.

5. Absorbierende Struktur (30, 38, 50, 58, 62, 66) nach einem oder mehreren der Ansprüche 1 bis 4, **dadurch gekennzeichnet, dass** der gewichtsprozentuale Anteil des thermoplastischen Polymers geringer als 30 Gew.% der absorbierenden Struktur ist.

6. Absorbierende Struktur (30, 38, 50, 58, 62, 66) nach Anspruch 5, **dadurch gekennzeichnet, dass** der gewichtsprozentuale Anteil des thermoplastischen Polymers geringer als 20 Gew.-% der absorbierenden Struktur ist.

7. Absorbierende Struktur (30, 38, 50, 58, 62, 66) nach Anspruch 6, **dadurch gekennzeichnet, dass** der gewichtsprozentuale Anteil des thermoplastischen Polymers geringer als 10 Gew.% der absorbierenden Struktur ist.

8. Absorbierende Struktur (30, 38, 50, 58, 62, 66) nach einem oder mehreren der vorstehenden Ansprüche, **dadurch gekennzeichnet, dass** das thermoplastische Polymer ein Polyolefin, insbesondere Polypropylen und/oder Polyethylen und/oder Ethylenvinylacetat (EVA), umfasst.

9. Absorbierende Struktur (30, 38, 50, 58, 62, 66) nach einem oder mehreren der vorstehenden Ansprüche, **dadurch gekennzeichnet, dass** der Schäumungsgrad wenigstens 20% beträgt.

10. Absorbierende Struktur (30, 38, 50, 58, 62, 66) nach einem oder mehreren der vorstehenden Ansprüche, **dadurch gekennzeichnet, dass** die Struktur als Zuschlagstoffe 3 - 20 Gew.-% , insbesondere 5 - 10 Gew.-%, an Fasern umfasst.

11. Absorbierende Struktur (38, 50, 58, 62, 66) nach einem oder mehreren der vorstehenden Ansprüche, **dadurch gekennzeichnet, dass** deren Flächengewicht in Längsrichtung und/oder Querrichtung variiert.

12. Absorbierende Struktur (30, 38, 50, 58, 62, 66) nach einem oder mehreren der vorstehenden Ansprüche, **dadurch gekennzeichnet, dass** eine oberflächenaktive Substanz als Zuschlagstoff zugesetzt ist.

13. Absorbierender Hygieneartikel zum einmaligen Gebrauch, insbesondere Windel, Damenbinde, Inkontinenzvorlage, mit einem insbesondere mehrschichtigen Saugkörper, **gekennzeichnet durch** eine Saugkörperlage aus einer absorbierenden Struktur (30, 38, 50, 58, 62, 66) nach einem oder mehreren der vorstehenden Ansprüche.

14. Hygieneartikel nach Anspruch 13, **dadurch gekennzeichnet, dass** die Saugkörperlage (70) auf der körperabgewandten Seite einer Flüssigkeitsverteiler- und Zwischenspeicherschicht (72) angeordnet ist.

15. Hygieneartikel nach Anspruch 14, **dadurch gekennzeichnet, dass** die Flüssigkeitsverteiler- und Zwischenspeicherschicht (72) ein thermoplastisches Polymer umfasst und unter Zusatz eines Treibmittels extrudiert ist.

16. Hygieneartikel nach Anspruch 15, **dadurch gekennzeichnet, dass** die Flüssigkeitsverteiler- und Zwischenspeicherschicht (72) keine superabsorbierenden Polymermaterialien umfasst.

17. Hygieneartikel nach Anspruch 15 oder 16, **dadurch gekennzeichnet, dass** die Flüssigkeitsverteiler- und Zwischenspeicherschicht (72) einen Schäumungsgrad von mehr als 20% aufweist.

18. Hygieneartikel nach Anspruch 15, 16 oder 17, **dadurch gekennzeichnet, dass** die Flüssigkeitsverteiler- und Zwischenspeicherschicht (72) als Zuschlagstoff 1 - 20 Gew.-%, insbesondere 5 - 15 Gew.-% Fasern enthält.

19. Hygieneartikel nach einem der Ansprüche 13-18 mit einer auf der körperabgewandten Seite der Saugkörperlage (70) vorgesehenen flüssigkeitsundurchlässigen Folienschicht (68), **dadurch gekennzeichnet, dass** die Folienschicht (68) zusammen mit der Saugkörperlage (70) extrudiert ist.

20. Hygieneartikel nach einem der Ansprüche 13 - 19, **dadurch gekennzeichnet, dass** der Saugkörper in Längsrichtung (44) des Artikels eine variierende Dicke aufweist.

21. Hygieneartikel nach einem der Ansprüche 13 - 20, **dadurch gekennzeichnet, dass** der Saugkörper in Querrichtung (40) des Artikels eine variierende Dicke aufweist.

22. Hygieneartikel nach einem oder mehreren der Ansprüche 13 - 21, **dadurch gekennzeichnet, dass** die Saugkörperlage beidseits in Längsrichtung (44) des Artikels verlaufende und in Richtung auf den Benutzer emporstehende Wandabschnitte (46) aufweist, welche eine Auslaufsperre bilden.

23. Hygieneartikel nach einem der Ansprüche 13 - 22, **dadurch gekennzeichnet, dass** die Saugkörperlage einen im wesentlichen in Querrichtung des Artikels verlaufenden und in Richtung auf den Benutzer emporstehenden Wandabschnitte aufweist.

24. Verfahren zum Herstellen einer absorbierenden Struktur nach einem oder mehreren der Ansprüche 1-12, die folgenden Verfahrensschritte umfassend:
- Einbringen eines thermoplastischen Polymers in eine Extrusionsvorrichtung,
- Einbringen eines superabsorbierenden körnigen Polymermaterials mit einem Feuchtigkeitsgehalt von wenigstens 0,5 Gew.-%, insbesondere wenigstens 1 Gew.-%, insbesondere von wenigstens 4 Gew.-%, in die Extrusionsvorrichtung,
- Schmelzen des thermoplastischen Polymermaterials bei Temperaturen unterhalb einer Schmelz- oder Zersetzungstemperatur des superabsorbierenden Polymermaterials,
- Extrudieren des Gemischs, wobei die Flüssigkeit in dem superabsorbierenden Polymermaterial bei Druckabbau verdampft und zur Schäumung des thermoplastischen Polymers führt, welches die körnigen Polymermaterialien miteinander matrixbildend verbindet.

25. Verfahren nach Anspruch 24, **dadurch gekennzeichnet, dass** der gewichtsprozentuale Anteil des superabsorbierenden Polymermaterials wenigstens 70 Gew.-% des in die Extrusionsvorrichtung eingebrachten Gemischs beträgt.

26. Verfahren nach Anspruch 24 oder 25, **dadurch gekennzeichnet, dass** das thermoplastische Polymer bei Temperaturen von 80 - 200 Grad Celsius erschmolzen wird.

27. Verfahren nach Ansprüche 24, 25 oder 26, **dadurch gekennzeichnet, dass** als Zuschlagstoff Fasern in die Extrusionsvorrichtung eingebracht werden.

28. Verfahren nach einem oder mehreren der Ansprüche 24 - 27, **dadurch gekennzeichnet, dass** als Zuschlagstoff eine oberflächenaktive Substanz in die Extrusionsvorrichtung eingebracht wird.

29. Verfahren nach einem oder mehreren der Ansprüche 25 - 28, **dadurch gekennzeichnet, dass** ein Extrusionsquerschnitt während des Extrudierens verändert wird.

30. Verfahren nach Anspruch 29, **dadurch gekennzeichnet, dass** der Extrusionsquerschnitt oszillierend verändert wird.

31. Verfahren nach einem oder mehreren der Ansprüche 24 - 30, **dadurch gekennzeichnet, dass** das Verfahren in einen Herstellungsprozess für Hygieneartikel integriert wird und dabei die absorbierende Struktur unmittelbar innerhalb einer schnellaufenden Herstellungsmaschine für Hygieneartikel extrudiert wird.

32. Verfahren nach Anspruch 31, **dadurch gekennzeichnet, dass** innerhalb der schnellaufenden Herstellungsmaschine ein zweischichtiger Saugkörper durch Coextrusion der Schichten gebildet wird, wobei der Saugkörper die absorbierende Struktur (70) als Saugkörperlage und eine auf deren körperzugewandten Seite vorgesehene Flüssigkeitsverteiler- und Zwischenspeicherschicht (72) umfasst.

33. Verfahren nach Anspruch 31 oder 32, **dadurch gekennzeichnet, dass** innerhalb der schnellaufenden Herstellungsmaschine ein dreischichtiger Saugkörper durch Coextrusion der Schichten gebildet wird, wobei die dritte Schicht eine flüssigkeitsundurchlässige Folienschicht (68) ist, die auf der körperabgewandten Seite der Saugkörperlage angeordnet ist.

## Claims

1. Absorbent structure (30, 38, 50, 58, 62, 66) formed on the basis of superabsorbent polymer materials, where the superabsorbent polymer materials are bonded together by a thermoplastic polymer, produced by extruding the thermoplastic polymer and superabsorbent polymer materials with a moisture content of at least 0.5 % by weight relative to the total mass of the superabsorbent polymer materials and thereby the fluid in the superabsorbent polymer materials is evaporated and foaming of the structure is induced.

2. Absorbent structure according to claim 1, **characterised in that** superabsorbent polymer materials having a moisture content of at least 1 % by weight are used for extrusion.

3. Absorbent structure according to claim 2, **characterised in that** superabsorbent polymer materials with a moisture content of at least 4 % by weight are used for extrusion.

4. Absorbent structure (30, 38, 50, 58, 62, 66) according to any one of the preceding claims, **characterised in that** the structure has a retention capacity of at least 10 g/g.

5. Absorbent structure (30, 38, 50, 58, 62, 66) according to one or more of claims 1 to 4, **characterised in that** the percentage by weight of the thermoplastic polymer is less than 30 % by weight of the absorbent structure.

6. Absorbent structure (30, 38, 50, 58, 62, 66) according to claim 5, **characterised in that** the percentage by weight of the thermoplastic polymer is less than 20 % by weight of the absorbent structure.

7. Absorbent structure (30, 38, 50, 58, 62, 66) according to claim 6, **characterised in that** the percentage by weight of the thermoplastic polymer is less than 10 % by weight of the absorbent structure.

8. Absorbent structure (30, 38, 50, 58, 62, 66) according to one or more of the preceding claims, **characterised in that** the thermoplastic polymer comprises a polyolefin, in particular polypropylene and/or polyethylene and/or ethylvinyl acetate (EVA).

9. Absorbent structure (30, 38, 50, 58, 62, 66) according to one or more of the preceding claims, **characterised in that** the degree of foaming is at least 20 %.

10. Absorbent structure (30, 38, 50, 58, 62, 66) according to one or more of the preceding claims, **characterised in that** the structure comprises 3 % to 20 % by weight, in particular 5 % to 10 % by weight, fibres as additives.

11. Absorbent structure (30, 38, 50, 58, 62, 66) according to one or more of the preceding claims, **characterised in that** its basis weight varies in the longitudinal and/or transverse direction.

12. Absorbent structure (30, 38, 50, 58, 62, 66) according to one or more of the preceding claims, **characterised in that** a surfactant substance is added as an additive.

13. Absorbent hygiene article for single use, in particular nappy, sanitary towel, incontinence pad, having in particular a multilayer absorbent core, **characterised by** an absorbent core layer from an absorbent structure (30, 38, 50, 58, 62, 66) according to one or more of the preceding claims.

14. Hygiene article according to claim 13, **characterised in that** the absorbent core layer (70) is located on the side of a fluid distribution and intermediate retention layer (72) facing away from the body.

15. Hygiene article according to claim 14, **characterised in that** the fluid distribution and intermediate retention layer (72) comprises a thermoplastic polymer and is extruded with the addition of a blowing agent.

16. Hygiene article according to claim 15, **characterised in that** the fluid distribution and intermediate retention layer (72) does not comprise any superabsorbent polymer materials.

17. Hygiene article according to claim 15 or claim 16, **characterised in that** the fluid distribution and intermediate retention layer (72) has a degree of foaming of more than 20 %.

18. Hygiene article according to claim 15, claim 16 or claim 17, **characterised in that** the fluid distribution and intermediate retention layer (72) contains as additive 1 % to 20 % by weight, in particular 5 % to 15 % by weight, fibres.

19. Hygiene article according to any one of claims 13 to 18 having a fluid impervious film (68) on the side of the absorbent core layer (70) facing away from the body, **characterised in that** the film (68) is extruded together with the absorbent core layer (70).

20. Hygiene article according to any one of claims 13 to 19, **characterised in that** the absorbent core has a varying thickness in the longitudinal direction (44) of the article.

21. Hygiene article according to any one claims 13 to 20, **characterised in that** the absorbent core has a varying thickness in the transverse direction (40) of the article.

22. Hygiene article according to one or more of claims 13 to 21, **characterised in that** the absorbent core layer has wall portions (46) on both sides running in the longitudinal direction (44) of the article and extending upward toward the wearer, which form a leakage barrier.

23. Hygiene article according to any one of claims 13 to 22, **characterised in that** the absorbent core layer has wall portions running substantially in the transverse direction of the article and extending upward toward the wearer.

24. Method for producing an absorbent structure according to one or more of claims 1 to 12, comprising the following steps:
- introduction of a thermoplastic polymer into an extrusion apparatus,
- introduction of a superabsorbent particulate polymer material having a moisture content of at least 0.5 % by weight, in particular at least 1 % by weight, particularly at least 4 % by weight, into the extrusion apparatus,
- melting the thermoplastic polymer material at temperatures below a melting or degradation temperature of the superabsorbent polymer material,
- extrusion of the mix, the fluid in the superabsorbent polymer material evaporating as pressure is reduced and resulting in foaming of the thermoplastic polymer, which bonds the particulate polymer materials together to create a matrix.

25. Method according to claim 24, **characterised in that** the percentage by weight of the superabsorbent polymer material is at least 70 % by weight of the mix introduced into the extrusion apparatus.

26. Method according to either claim 24 or claim 25, **characterised in that** the thermoplastic polymer becomes molten at temperatures of 80 to 200°C.

27. Method according to claim 24, claim 25 or claim 26, **characterised in that** fibres are introduced as an additive into the extrusion apparatus.

28. Method according to one or more of claims 24 to 27, **characterised in that** a surfactant substance is introduced as an additive into the extrusion apparatus.

29. Method according one or more of claims 25 to 28, **characterised in that** an extrusion cross-section is changed during extrusion.

30. Method according to claim 29, **characterised in that** the extrusion cross-section is changed in an oscillating fashion.

31. Method according to one or more of claims 24 to 30, **characterised in that** the method is integrated into a production process for hygiene articles and therein the absorbent structure is extruded directly inside machinery for the high-speed production of hygiene articles.

32. Method according to claim 31, **characterised in that**, inside the high-speed production machinery, a double-layer absorbent core is formed by co-extrusion of the layers, the absorbent core comprising the absorbent structure (70) as an absorbent core layer and a fluid distribution and intermediate retention layer (72) on the side of the core layer facing the body.

33. Method according to either claim 31 or claim 32, **characterised in that**, inside the high-speed production machinery, a triple-layer absorbent core is formed by co-extrusion of the layers, the third layer being a fluid-impervious film (68), which is located on the side of the absorbent core layer facing away from the body.

## Revendications

1. Structure absorbante (30, 38, 50, 58, 62, 66) formée à partir de matériaux polymères superabsorbants, les matériaux polymères superabsorbants étant reliés entre eux par un polymère thermoplastique, fabriquée en ce que des matériaux polymères superabsorbants ayant un taux d'humidité d'au moins 0,5 % en poids rapporté à la masse totale des matériaux polymères superabsorbants et le polymère thermoplastique sont extrudés, le liquide des matériaux polymères superabsorbants s'évaporant alors et un agent de moussage étant amené dans la structure.

2. Structure absorbante selon la revendication 1, **caractérisée en ce que** des matériaux polymères superabsorbants ayant un taux d'humidité d'au moins 1 % en poids sont utilisés pour l'extrusion.

3. Structure absorbante selon la revendication 2, **caractérisée en ce que** des matériaux polymères superabsorbants ayant un taux d'humidité d'au moins 4 % en poids sont utilisés pour l'extrusion.

4. Structure absorbante (30, 38, 50, 58, 62, 66) selon l'une des revendications précédentes, **caractérisée en ce que** la structure présente une capacité de rétention d'au moins 10 g/g.

5. Structure absorbante (30, 38, 50, 58, 62, 66) selon l'une ou plusieurs des revendications 1 à 4, **caractérisée en ce que** la part en pourcentage en poids du polymère thermoplastique est inférieure à 30 % en poids de la structure absorbante.

6. Structure absorbante (30, 38, 50, 58, 62, 66) selon la revendication 5, **caractérisée en ce que** la part en pourcentage en poids du polymère thermoplastique est inférieure à 20 % en poids de la structure absorbante.

7. Structure absorbante (30, 38, 50, 58, 62, 66) selon la revendication 6, **caractérisée en ce que** la part en pourcentage en poids du polymère thermoplastique est inférieure à 10 % en poids de la structure absorbante.

8. Structure absorbante (30, 38, 50, 58, 62, 66) selon l'une ou plusieurs des revendications précédentes, **caractérisée en ce que** le polymère thermoplastique comprend une polyoléfine, en particulier du polypropylène et/ou du polyéthylène et/ou de l'éthylène-acétate de vinyle (EVA).

9. Structure absorbante (30, 38, 50, 58, 62, 66) selon l'une ou plusieurs des revendications précédentes, **caractérisée en ce que** le taux de moussage est d'au moins 20 %.

10. Structure absorbante (30, 38, 50, 58, 62, 66) selon l'une ou plusieurs des revendications précédentes, **caractérisée en ce que** la structure comprend comme adjuvants de 3 à 20 % en poids, en particulier de 5 à 10 % en poids de fibres.

11. Structure absorbante (30, 38, 50, 58, 62, 66) selon l'une ou plusieurs des revendications précédentes, **caractérisée en ce que** son poids par unité de surface varie dans le sens longitudinal et/ou transversal.

12. Structure absorbante (30, 38, 50, 58, 62, 66) selon l'une ou plusieurs des revendications précédentes, **caractérisée en ce qu'**une substance tensioactive est ajoutée comme adjuvant.

13. Article d'hygiène absorbant à usage unique, en particulier couche, serviette hygiénique, protection pour incontinence, comprenant un corps absorbant en particulier à plusieurs couches, **caractérisé par** une couche de corps absorbant constituée d'une structure absorbante (30, 38, 50, 58, 62, 66) selon l'une ou plusieurs des revendications précédentes.

14. Article d'hygiène selon la revendication 13, **caractérisé en ce que** la couche de corps absorbant (70) est agencée sur la face opposée au corps d'une couche de répartition et de rétention intermédiaire de liquide (72).

15. Article d'hygiène selon la revendication 14, **caractérisé en ce que** la couche de répartition et de rétention intermédiaire de liquide (72) comprend un polymère thermoplastique et est extrudée en ajoutant un agent gonflant.

16. Article d'hygiène selon la revendication 15, **caractérisé en ce que** la couche de répartition et de rétention intermédiaire de liquide (72) ne comprend pas de matériaux polymères superabsorbants.

17. Article d'hygiène selon la revendication 15 ou 16, **caractérisé en ce que** la couche de répartition et de rétention intermédiaire de liquide (72) présente un taux de moussage de plus de 20 %.

18. Article d'hygiène selon la revendication 15, 16 ou 17, **caractérisé en ce que** la couche de répartition et de rétention intermédiaire de liquide (72) contient comme adjuvant de 1 à 20 % en poids, en particulier de 5 à 15 % en poids de fibres.

19. Article d'hygiène selon l'une des revendications 13 à 18 comprenant une couche de film (68) imperméable aux liquides prévue sur la face opposée au corps de la couche de corps absorbant (70), **caractérisé en ce que** la couche de film (68) est extrudée conjointement avec la couche de corps absorbant (70).

20. Article d'hygiène selon l'une des revendications 13 à 19, **caractérisé en ce que** le corps absorbant présente une épaisseur variant dans le sens longitudinal (44) de l'article.

21. Article d'hygiène selon l'une des revendications 13 à 20, **caractérisé en ce que** le corps absorbant présente une épaisseur variant dans le sens transversal (40) de l'article.

22. Article d'hygiène selon l'une des revendications 13 à 21, **caractérisé en ce que** la couche de corps absorbant présente des segments de paroi (46) formant une barrière à l'écoulement qui s'étendent de chaque côté dans le sens longitudinal (44) de l'article et s'élèvent en direction de l'utilisateur.

23. Article d'hygiène selon l'une des revendications 13 à 22, **caractérisé en ce que** la couche de corps absorbant présente un segment de paroi s'étendant essentiellement dans le sens transversal de l'article et s'élevant en direction de l'utilisateur.

24. Procédé pour la fabrication d'une structure absorbante selon l'une ou plusieurs des revendications 1 à 12, comprenant les étapes de procédé suivantes :
- chargement d'un polymère thermoplastique dans un dispositif d'extrusion,
- chargement dans le dispositif d'extrusion d'un matériau polymère superabsorbant en grains ayant un taux d'humidité d'au moins 0,5 % en poids, en particulier d'au moins 1 % en poids, en particulier d'au moins 4 % en poids,
- fusion du matériau polymère thermoplastique à des températures situées en-dessous d'une température de fusion ou de décomposition du matériau polymère superabsorbant,
- extrusion du mélange, le liquide s'évaporant dans le matériau polymère superabsorbant lors de la réduction de pression et conduisant au moussage du polymère thermoplastique qui lie les matériaux polymères en grains entre eux en formant une matrice.

25. Procédé selon la revendication 24, **caractérisé en ce que** la part en pourcentage en poids du matériau polymère superabsorbant est d'au moins 70 % en poids du mélange chargé dans le dispositif d'extrusion.

26. Procédé selon la revendication 24 ou 25, **caractérisé en ce que** le polymère thermoplastique est fondu à des températures comprises entre 80 et 200 degrés Celsius.

27. Procédé selon les revendications 24, 25 ou 26, **caractérisé en ce que** des fibres sont introduites dans le dispositif d'extrusion comme adjuvant.

28. Procédé selon l'une ou plusieurs des revendications 24 à 27, **caractérisé en ce qu'**une substance tensioactive est introduite dans le dispositif d'extrusion comme adjuvant.

29. Procédé selon l'une ou plusieurs des revendications 25 à 28, **caractérisé en ce qu'**une section transversale d'extrusion est modifiée pendant l'extrusion.

30. Procédé selon la revendication 29, **caractérisé en ce que** la section transversale d'extrusion est modifiée de manière oscillante.

31. Procédé selon l'une ou plusieurs des revendications 24 à 30, **caractérisé en ce que** le procédé est intégré dans un processus de fabrication d'articles d'hygiène dans lequel la structure absorbante est extrudée directement à l'intérieur d'une machine de fabrication d'articles d'hygiène à cadence rapide.

32. Procédé selon la revendication 31, **caractérisé en ce que**, à l'intérieur de la machine de fabrication à cadence rapide, un corps absorbant à deux couches est formé par coextrusion des couches, le corps absorbant comprenant la structure absorbante (70) en tant que couche de corps absorbant et une couche de répartition et de rétention intermédiaire de liquide (72) prévue sur sa face orientée vers le corps.

33. Procédé selon la revendication 31 ou 32, **caractérisé en ce que**, à l'intérieur de la machine de fabrication à cadence rapide, un corps absorbant à trois couches est formé par coextrusion des couches, la troisième couche étant une couche de film imperméable aux liquides (68) qui est agencée sur la face de la couche de corps absorbant opposée au corps.
